# EUROPEAN PATENT SPECIFICATION

(11) **EP 4 165 029 B1**
(45) Date of publication and mention of the grant of the patent: **17.07.2024**
(21) Application number: 21731202.4
(22) Date of filing: 15.06.2021
(51) Int. Cl.: C07D 277/44, A61P 17/00, A61K 31/426

(54) **PROCESS FOR THE MANUFACTURE OF ALKYLAMIDOTHIAZOLES**
VERFAHREN ZUR HERSTELLUNG VON ALKYLAMIDOTHIAZOLEN
PROCÉDÉ DE FABRICATION D'ALKYLAMIDOTHIAZOLES

(30) Priority: 15.06.2020 EP 20180056; 15.06.2020 EP 20180053
(43) Date of publication of application: 19.04.2023
(73) Proprietor: DSM IP Assets B.V., 6411 TE Heerlen (NL)
(72) Inventor: GLAUS, Florian David, 4303 Kaiseraugst (CH); HEIDL, Marc, 4303 Kaiseraugst (CH); SCHLIFKE-POSCHALKO, Alexander, 4303 Kaiseraugst (CH)
(74) Representative: dsm-firmenich IP
(86) International application number: PCT/EP2021/066077
(87) International publication number: WO 2021/255011

(56) References cited:
- EP-A2- 1 709 964
- WO-A1-2015/090850

## Description

The invention relates to an improved process for the manufacture of alkylamidothiazoles. This novel economical process provides products in high purity and yields.

Alkylamidothiazoles are highly effective skin lightening agents which are, for example, used for the treatment and/or prophylaxis of undesired skin pigmentation. Furthermore, they have been described as antioxidants or free-radical scavengers. Thus, alkylamidothiazoles such as in particular thiamidol [CAS No. 1428450-95-6] are highly interesting compounds for the cosmetic industry.

Thiamidol is prepared in a multi-step synthesis starting from 2,4-dihydroxyacetophenone as outlined in US 2014/0121250 A1. The reaction scheme includes the introduction of protecting groups to suppress unwanted side reactions of the phenolic hydroxyl groups, as well as a bromination step. The use of protection groups, however, increases the cost, while bromination, next to being a cost driver, is also preferably avoided in industrial processes due to the toxicity, environmental hazards and corrosiveness of bromine.

EP1709964 discloses the reaction of 2-chloro-1-(2,4-dihydroxyphenyl)ethan-1-one in methanol using an excess of base leading to the formation of the corresponding 6-hydroxybenzofuran-3(2H)-one.

Because of the continuously increasing demand for thiamidol, the object of the present invention was to provide an improved process for the preparation of alkylamidothiazoles which is scalable, easy to carry out and affords economic advantages as a result of a short synthesis and high yields. Furthermore such process should not or only to a very little extend lead to the formation of the respective 6-hydroxybenzofuran-3(2*H*)-one as it cannot not readily separated from thiamidol, in particular in an economical manner and in commercial (large) scale processes.

Surprisingly, it has now been found that the use of protection groups as disclosed in US 2014/0121250 is not necessary if no base is added to the reaction, or the base is dosed such to solely quench the acid formed during the reaction. Furthermore, bromination can be avoided as 2-chloro-1-(2,4-dihydroxyphenyl)ethanone (formula (II)) can readily be used as starting material. WO2015/090850 discloses a process for the preparation of alkylamidothiazoles whereby the hydroxy groups of the starting material were protected.

Thus, the invention relates to a process for the preparation of compounds of formula (I) 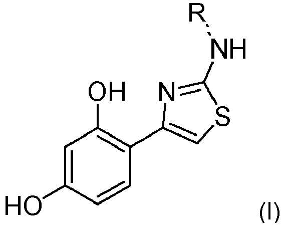 wherein
R is selected from the group of H or C(=O)R' with R' being selected from the group of C₁₋₁₂ alkyl, hydroxyC₁₋₁₂ alkyl, C₁₋₄ alkoxyC₁₋₁₂ alkyl, aminoC₁₋₁₂ alkyl, C₂₋₁₂ alkenyl, C₃₋₈cycloalkyl, hydroxyC₁₋₃alkylC₃₋₈cycloalkyl and p-hydroxyC₁₋₃ alkylarylC₀₋₃ alkyl
said process comprising the step of reacting a compound of formula (II) with a thiourea of formula (III) 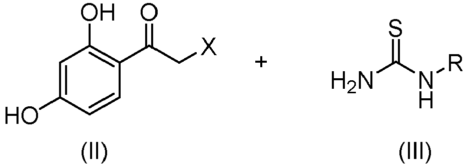
wherein
R is as defined above and
X is a leaving group selected from Cl, Br, OTs and OMs (which step is also referred herein as step-1).

It is the surprising finding of the present invention that, contrast to the prior art processes said reaction can be carried out either in the absence of a base, which is preferred or a base is continuously added to quench the acid HX formed during the reaction, preferably after all reactants have been added and the reaction mixture has been heated, most preferably to reflux.

In all embodiments of the present invention preferably X is CI.

In all embodiments, preferably R is C(=O)R', with all the definitions as given above.

Thus, in a particularly preferred embodiment, the present invention relates to a process for the preparation of compounds of formula (Ia) 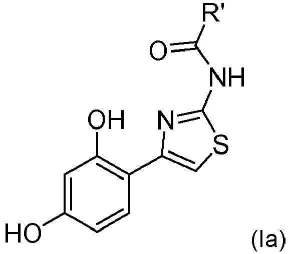 wherein
R' is selected from the group consisting of C₁₋₁₂ alkyl, hydroxyC₁₋₁₂ alkyl, C₁₋₄ alkoxyC₁₋₁₂ alkyl, aminoC₁₋₁₂ alkyl, C₂₋₁₂ alkenyl, C₃₋₈cycloalkyl, hydroxyC₁₋₃alkylC₃₋₈acycloalkyl and p-hydroxyC₁₋₃ alkylarylC₀₋₃ alkyl
said process comprising the step of reacting 2-chloro-1-(2,4-dihydroxyphenyl)ethanone (Ila) with an acylthiourea of formula (Illa) 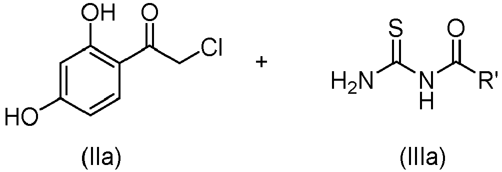
wherein
R' is as defined above.

In the present document, the term "C_{x-y}" refers to a hydrocarbon residue comprising x to y carbon atoms, which residue may be linear (straight chain) or branched.

In the present document (m)ethyl or (m)ethoxy refers to the respective methyl or ethyl derivative.

The term OTs refers to a tosylate leaving groups and the term OMs refers to a mesylate leaving group, both of which are well known to a person skilled in the art.

The term C₁₋₁₂ alkyl groups refers to unbranched C₁-C₁₂alkyl, respectively branched C₃-C₁₂alkyl groups, such as e.g. particular methyl, ethyl, *n*-propyl, *i*-propyl or *i*-, *n-,* secondary or *t*-butyl and n-hexyl groups. Preferably, in all embodiments of the present invention, the C₁₋₁₂ alkyl group is a C₁₋₆ alkyl group, such as more preferably methyl, ethyl, propyl, i-propyl, t-butyl or n-hexyl and most preferably isopropyl.

The term "hydroxyC₁₋₁₂ alkyl" is used herein to refer to alkyl groups as defined above, wherein one hydrogen atom is replaced by a hydroxy group such as e.g. hydroxy(m)ethyl, hydroxypropyl, hydroxybutyl, hydroxypentyl and hydroxyhexyl. Preferably, the hydroxy group is terminal, i.e. the hydroxyC₁₋₁₂ alkyl group is a -(CH₂)ₙ-OH group with n being an integer from 1 to 12, preferably from 1 to 8, most preferably from 1 to 5. Preferably, in all embodiments of the present invention, the hydroxyC₁₋₁₂ alkyl group is a hydroxyC₁₋₅ alkyl group, such as most preferably 2-hydroxyethyl (-(CH₂)₂-OH) or 5-hydroxybutyl (-(CH₂)₅-OH).

The term "C₁₋₄ alkoxyC₁₋₁₂ alkyl" is used herein to refer to alkyl groups as defined above, wherein one hydrogen atom is replaced by an C₁₋₄alkoxy group, preferably by a (m)ethoxy group. Exemplary C₁₋₄ alkoxyC₁₋₁₂ alkyl groups encompass (m)ethoxymethyl, (m)ethoxyethyl, (m)ethoxypropyl, (m)ethoxybutyl, (m)ethoxypentyl and (m)ethoxyhexyl, Preferably, the C₁₋₄ alkoxy group is terminal, i.e. the C₁₋₄ alkoxyC₁₋₁₂ alkyl group is a -(CH₂)ₙ-OC₁₋₄ alkyl group with n being an integer from 1 to 12, preferably from 1 to 8, most preferably from 1 to 5. Preferably, in all embodiments of the present invention, the C₁₋₄ alkoxyC₁₋₁₂ alkyl group is a (m)ethoxyC₁₋₅alkyl, such as most preferably 2-methoxyethyl.

The term "aminoC₁₋₁₂alkyl" is used herein to refer to alkyl groups as defined above, wherein one hydrogen atom is replaced by an amino group such as amino(m)ethyl, aminopropyl, aminobutyl, aminopentyl and aminohexyl. Preferably, the amino group is terminal, i.e. the aminoC₁₋₁₂ alkyl group is a -(CH₂)ₙ-NH₂ group with n being an integer from 1 to 12, preferably from 1 to 8, most preferably from 1 to 5. Preferably, in all embodiments of the present invention, the aminoC₁₋₁₂ alkyl group is a aminoC₁₋₅ alkyl group such as in particular a 2-aminoethyl (-(CH₂)₂-NH₂) or a 5-aminobutyl (-(CH₂)₅-NH₂) group.

The term "C₂₋₁₂ alkenyl" as used herein refers to monovalent straight or branched alkyl chains which have at least one carbon-carbon double bond, which may be (independently from each other) in (E) or (Z)-configuration. In one embodiment alkenyl is C₂₋₁₂alkenyl, in another embodiment, C₂₋₆alkenyl, in another embodiment C₂₋₄alkenyl. Preferably, in all embodiments of the present invention, the alkenyl group is selected from the group of linear (straight chain) alkenyl groups, such as in particular linear C₂₋₄alkenyl groups.

The term "C₃₋₈cycloalkyl" is used herein to refer to monovalent, saturated, linear or branched cyclic hydrocarbyl groups comprising 3 to 8 carbon atoms such as e.g. cyclopentyl, cyclohexyl, cycloheptyl, 2,5-dimethylcyclopentyl and 2,6-dimethylcyclohexyl. Preferred C₃₋₈cycloalkyl in all embodiments according to the present invention are cyclopentyl and cyclohexyl.

The term "hydroxyC₁₋₃alkylC₃₋₈cycloalkyl" as used herein refers to cycloalkyl groups as defined above, which are substituted with a hydroxyC₁₋₃alkyl group as defined above. Exemplary and particular advantageous hydroxyC₁₋₃alkylC₃₋₈cycloalkyl groups are e.g. (hydroxy(m)ethyl)cyclopentyl or (hydroxy(m)ethyl)cyclohexyl. Most preferred In all embodiments according to the present invention is 4-(hydroxymethyl)cyclohexyl.

The term "aryl" as used herein refers to an aromatic substituent containing 5 to 15 carbon atoms and containing a single aromatic ring or multiple aromatic rings which are fused together, directly linked or indirectly linked (such that the different aromatic rings are bound to a common group such as a methylene or ethylene group). Particularly advantageous aryl groups according to the present invention contain 5 to 12 carbon atoms containing a single aromatic ring or multiple aromatic rings which are fused together. Exemplary aryl groups are phenyl and naphtyl groups. The most preferred aryl residue in all embodiments of the present invention is phenyl.

The term "alkylene" is used herein to refer to divalent saturated, linear or branched hydrocarbon groups. Exemplary alkylene groups include methylene, ethylene or propylene.

The term "p-hydroxyC₁₋₃ alkylarylCₒ₋₃ alkyl" as used herein refers to aryl groups as defined above which are substituted with a hydroxyC₁₋₃alkyl group and which are linked via a C₀₋₃ alkylene group to the amid moiety. Exemplary and particular advantageous p-hydroxyC₁₋₃ alkylarylCₒ₋₃ alkyl groups are e.g. (p-hydroxy(m)ethylphenyl)(m)ethyl. Most preferred In all embodiments according to the present invention is p-(hydroxymethylphenyl)methyl.

Preferably in all embodiments of the present invention, R is C(=O)R' with R' being a C₁₋₈ alkyl, more preferably a C₂₋₆ alkyl, most preferably isopropyl.

Most in particular, the invention relates to a process for the preparation of thiamidol (i.e. a compound of formula (I) wherein R is C(=O)R' and R' is isopropyl.

In case identical labels for symbols or groups are present in several formulae, in the present document, the definition of said group or symbol made in the context of one specific formula applies also to other formulae which comprises the same said label.

It is well understood, that all definitions for the compound of formula (I), (II) and (III) as far as reasonable applicable, also apply for the compounds of formula (Ia), (IIa) and (IIIa).

The compounds of formula (I) can be provided either in the form of a free base, or in the form of a cosmetically acceptable salt thereof.

The term 'or a cosmetically acceptable salt thereof' preferably refers to compounds of formula (I) in the form of an acid addition salt such as in the form of a chloride, a bromide, an iodide, a sulphate, a phosphate, an ascorbate an acetate, a mesylate, a tosylate or a trifluoroacetate salt. Particularly suitable are the respective chloride and bromide salts, preferably the hydrochlorides.

The process according to the present invention is advantageously carried out in an inert solvent.

The term inert solvent as used herein means a solvent which does not react under the given reaction conditions.

Particular suitable inert solvents to be used in the process according to the present invention, i.e. in step-1 are selected from the group consisting of
- water;
- alkyl alcohols such as methanol, ethanol, 1-propanol, isopropanol, 1-butanol, 2-butanol, preferably methanol, ethanol and isopropanol; most preferably ethanol;
- ethers such as tetrahydrofuran, 2-methyltetrahydrofuran, dioxane, diethyl ether, methyl t-butyl ether (MTBE), cyclopentyl methyl ether (CPME), 1,2-dimethoxyethane, bis(2-methoxyethyl) ether, preferably tetrahydrofuran, 2-methyltetrahydrofuran, dioxane and cyclopentyl methyl ether, more preferably dioxane and cyclopentyl methyl ether; most preferably cyclopentyl methyl ether;
- ketones such as acetone;
- dipolar aprotic solvents such as acetonitrile, N,N-dimethylformamide and N-methyl pyrrolidinone; and
- aromatic hydrocarbon solvents such as toluene or xylene
as well as any mixtures thereof, the alkyl alcohols and ethers being the preferred class of inert solvents to be used in the process of the present invention (i.e. in step-1).

Advantageous inert solvents in the process according to the present invention, i.e. in step-1 are ethanol, acetonitrile, acetone, dioxane and cyclopentyl methyl ether, optionally in admixture with water. In said admixture, the amount of water (volume) is preferably selected in the range from 0.1 to 25 %, preferably from 1 to 20%, most preferably in the range of 5 to 15 %.

Most preferred in all embodiments of the present invention is the use of ethanol, a mixture of ethanol and water, dioxane, dioxane and water, or cyclopentyl methyl ether, most preferably dioxane or cyclopentyl methyl ether, cyclopentyl methyl ether being the most preferred solvent.

In one particular advantageous embodiment, the solvent in step-1 is dioxane or cyclopentyl methyl ether as these solvent can already be used in the previous step, i.e. in the preparation of the compound of formula (II) such as in particular of formula (Ila), which in said case does not need to be isolated resulting in further overall improved yields.

The reaction temperature in the process according to the present invention, i.e. in step-1 is advantageously at least 40°C, preferably at least 50°C. More preferably, the reaction temperature is selected in the range from 40 C to 150°C such as more preferably in the range from 50°C to 110°C, even more preferably in the range from 60° to 110°C, such as most preferably in the range of 70 to 110°C and/ or the reaction is carried out at reflux.

Pressure is not critical to the present process, except to the extent that the selection of a particular pressure may lower the boiling point.

The compounds of formula (II) and (III) in the process according to the present invention, i.e. in step-1 are advantageously used in equimolar amounts, alternatively however, one may also be used in a slight excess. Then the molar ratio of the two compounds is preferably selected in the range of 1.25 to 1, such as more in particular in the range from 1.1 to 1 such as e.g. in the range from 1.05 to 1. Particularly, compound of formula (III) may be used in a slight excess in the process according to the present invention. In such cases, the molar ratio of the compound of formula (III) to the compound of formula (II) is selected in the range from about 1.5 to 1 such as more in particular in the range from 1.25 to 1 such as e.g. in the range from about 1.15 to 1.

Preferably, in all embodiments of the present invention an inert solvent with all the definitions and preferences as given herein is present in step-1. The amount of the inert solvent(s) is preferably selected in the range from 0.25 to 15 mL/mmol of the compound of formula (II), preferably in the range from 0.5 to 10mL/mmol of the compound of formula (II), most preferably in the range from 1 to 5mL/mmol of the compound of formula (II). Further suitable ranges encompass 0.25 to 10 mL/mmol of the compound of formula (II), more preferably in the range from 0.35 to 4 mL/mmol of the compound of formula (II), most preferably in the range from 0.5 to 2mL/mmol of the compound of formula (II).

According to a preferred embodiment according to the invention in step-1 a reaction mixture is provided that contains the compounds of formula (II) and (III) and an inert solvent as defined herein. Then the reaction is heated up to the reaction temperature with all the definitions and preferences as given above.as defined above. Preferably, the addition of the base is only started after the reaction mixture has been heated up to at least 40°C, preferably at least 50°C, most preferably to the reaction temperature.

During the reaction in step-1, 1 mol equivalent of acid is formed. However, if base is added as suggested in US 2014/0121250 A1, substantial amounts of the respective 3-coumaranone derivative such as e.g. 6-hydroxybenzofuran-3(2*H*)-one are formed as a side product as also shown in the comparative examples contained herein.

Surprisingly, though, it was found that under the conditions outlined herein high conversions in step-1 are achieved in the absence of base. Thus, in one preferred aspect of the invention, the process according to the present invention (i.e. step-1) is carried out in the absence of a base.

The reaction time in the process without base according to the present invention, i.e. in step-1 is preferably selected in the range from 3 h to 24 h, more preferably in the range from 5 h to 22 h, most preferably in the range 6 h to 20 h.

In another aspect of the invention, however, a base is slowly/continuously added in step-1, preferably under pH control, in order to neutralize the acid such as e.g. HCl generated by the reaction. The base is dosed such, that the acid is quenched as formed, more preferably after all reactants (i.e. the compound of formula (II and III)) and optionally the inert solvent are added and the thus obtained reaction mixture has been heated to the rection temperature, preferably to reflux.

The dosage is furthermore advantageously adjusted such that the pH of the reaction mixture is maintained below pH 5.5, preferably below 5 as this leads to particular good yields. Furthermore it is advantageous in all embodiments of the present invention that the lower pH is at or over a pH 2, preferably at or over pH 3.

Even more preferably, in all embodiments of the present invention, the pH in step-1 is adjusted such, that it is maintained in the range from 2 to 5.5, preferably in the range from 3 to 5.5, most preferably in the range from 3 to 5 during the reaction.

It is well understood, that no free base may be present right from the beginning of the reaction as then high amounts of the respective 3-cumaranone derivatives are formed.

The reaction time in the process with base according to the present invention, i.e. in step-1 is preferably selected in the range from 30 min to 24 h, more preferably in the range from 1 h to 12 h, most preferably in the range 1 h to 6 h.

Suitable bases encompass NaHCOs, KHCOs, Na₂CO₃, K₂CO₃, LiOH, NaOH, KOH, NaOMe, NaOEt, NaOEt, NaOMe, NaOAc, KOAc. The base can be added in solid form or as aqueous or alcoholic solution thereof, such as e.g. in the form of an ethanolic NaOMe and/or NaOEt solution or aqueous NaHCO₃ solution.

In another embodiment suitable bases to be used in step-1 encompass triethylamine, N,N-diisopropylethylamine, piperidine, pyrrolidine, morpholine, 4-methylmorpholine, 4-ethylmorpholine, 1,3-diazabicyclo[2.2.2.]octane), 2,6-lutidine, pyridine. More preferred bases in step-1 according to the present invention tertiary amine bases such as triethylamine, diisopropylethylamine, 4-methylmorphoine, 4-ethylmorpholine, most preferred are N,N-diisopropylethylamine and/ or 4-methylmorpholine.

The total amount of base, if any, to be dosed into the reaction mixture is preferably selected in the range from 0.1 to 1.5 mole equivalents, preferably in the range from 0.25 to 1.25 mole equivalents, most preferably in the range from 0.5 to 1.15 mole equivalents, based on the compound of formula (II). Further suitable ranges encompass 0.5 to 1.15 mole equivalent, 0.5 to 1.1 mole equivalents, 0.25 to 1.1 mole equivalents, 0.25 to 1.05 mole equivalents, 0.5 to 1.05 mole equivalents, 0.75 to 1.05 mole equivalents, 0.25 to 1.0 mole equivalents, 0.5 to 1.0 mole equivalents, and 0.75 to 1.0 mole equivalents.

After the reaction of step-1 is complete, the mixture is cooled, preferably to ambient or lower temperatures such as e.g. temperatures in the range from 0 to 25 °C followed by the addition of water or aqueous solutions of an acid or a base. In the case of thiamidol, upon addition of water or aqueous acid a precipitate is formed which can subsequently be isolated by filtration.

If step-1 is performed in the absence of water and/ or an alcoholic solvent, the compound of formula (I) is either precipitating directly from the reaction mixture or may be precipitated by the addition of anhydrous acid, such as e.g. HCl in CPME or dioxane which allows the isolation by filtration without the addition of water, which is a preferred embodiment of the present invention.

In a further particular embodiment of the invention, using a solvent immiscible with water such as CPME, thiamidol may also be isolated by extraction into an aqueous phase at basic pH followed by precipitation by pH adjustment (acidifying) of said aqueous phase after removal of the organic layer.

Thus, in a preferred embodiment the process of the present invention furthermore comprises a step-2, following step-1, which consists of a step-2a: basifying the obtained reaction to mixture to pH 12-14, preferably 12.5 to 14 followed by extraction of the thus obtained salt of the compound of formula (I) into water (step-2b) and subsequent acidifying of the aqueous phase to pH 2-10, more preferably pH 3-8, most preferably pH 4-7 (step-2c), resulting in the precipitation of the compound of formula (I). In said precipitation step (step-2c), a water miscible solvent is preferably added to aid precipitation. Preferred water miscible solvents are EtOH, MeOH or iPrOH, most preferred is EtOH. The ratio between water and said water miscible solvent may be selected from 1:1 to 4:1, preferably from 1.5:1 to 3:1. Suitable acids for the acidification step-2c encompass HCl, H₃PO₄, acetic acid, NaH₂PO₄, Na₂HPO₄, KH₂PO₄, K₂HPO₄, H₂SO₄, NaHSO₄, KHSO₄, most preferred are HCl, acetic acid and H₃PO4.

In all embodiments of the present invention, preferably the compound of formula (II) is 2-chloro-1-(2,4-dihydroxyphenyl)ethanone (i.e. the compound of formula (Ila) [CAS No. 25015-92-3] .

2-Chloro-1-(2,4-dihydroxyphenyl)ethanone can be prepared according to known processes in the art. Preferably 2-chloro-1-(2,4-dihydroxyphenyl)ethanone is prepared from resorcinol as this is readily available at low costs.

Thus, in a further advantageous embodiment of the present invention, the process of the present invention encompasses the step of preparing 2-chloro-1-(2,4-dihydroxyphenyl)ethanone from resorcinol (IV) and chloroacetonitrile (V) in the presence of a Lewis acid (Houben Hoesch reaction), e.g. as outlined below and disclosed in US 2007/0265332 A1 (step-0).

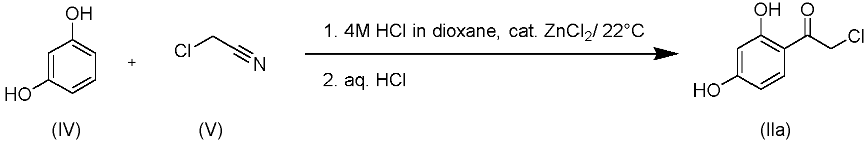

Alternatively, the compound of formula (II) can be prepared by Friedel Crafts acylation of resorcinol and chloroacetylchlorid as e.g. outlined in WO 2000/031588 A1.

In a particularly advantageous embodiment the compound of formula (Ila) is prepared in a process encompassing the step of reacting resorcinol with chloroacetonitrile in cyclopentyl methyl ether ether and/ or dioxane as then the compound of formula (Ila) does not need to be isolated, but can be used as a solution of a compound of formula (Ila) in cyclopentyl methyl ether or dioxane in the subsequent reaction step further improving the overall yield.

Thus in a particular advantageous embodiment, the present invention relates to a process for the preparation of compounds of formula (Ia), said process comprising the consecutive steps of

| | |
|---|---|
| Step-0(1): | reacting resorcinol (IV) with chloroacetonitril (V) (Houben Hoesch reaction) in a solvent selected from cyclopentyl methyl ether or dioxane, followed by |
| Step-0(2) | hydrolysis of the resulting reaction mixture to obtain the compound of formula (Ila), followed by |
| Step-0(3) | aqueous work-up and optional azeotropic drying to obtain an organic phase comprising the solvent and the compound of formula (Ila), followed by |
| Step-1(1): | addition of an acylthiourea of formula (Illa) to the organic phase obtained in step-0(3). |

The Lewis acid in step-0(1) can be selected from the group consisting of AlCl₃ and ZnCl₂. Preferably, the Lewis acid is AlCl₃. The amount of the Lewis acid in step-0(1) is preferably selected in the range from 0.25-1.5 eq with respect to resorcinol, more preferably in the range from 0.8-1.2 eq, most preferably from 0.9-1.2 eq. (for AlCl₃), respectively 0.3-0.7 eq. for ZnCl₂.

The amount of HCl initially present in step-0(1) is generally selected in the range from 1-10 eq. with respect to resorcinol, more preferably from of 2-6 eq., most preferably from 2-4 eq.

In another embodiment of the invention, additional gaseous HCl is added after all components required in step-0(a) have been added to the reaction mixture, either continuously or in portions.

The temperature in step-0(1) is preferably selected in the range from -10 C to 40 °C, more preferably from 0 to 25 °C.

The hydrolysis (step-0(2)) is performed, preferably, by adding water or diluted aqueous acid, e.g. HCl, to the reaction mixture, more preferably water, followed by heating of the thus obtained reaction mixture. The addition is preferably carried out in way such that the temperature is maintained in the range from 0 °C to 90 °C, more preferable from 0 °C to 60 °C, most preferably from 0 °C to 40 °C. After the addition is completed, the actual hydrolysis is performed at a temperature of 40-110 °C, more preferably of 60-100 °C, most preferably of 70-95 °C.

It is well understood, that all the conditions and preferences with regard to temperature, amounts, ratios etc. as outlined herein for step-1 also apply to step-1(1).

The compounds of formula (III) can be prepared as outlined in US 2014/0121250 A1.

In all embodiments of the present invention, the compound of formula (III) is preferably N-carbamothioylisobutyramide [CAS No. 6965-58-8, also known as N-isobutyrylthiourea].

Each reaction of the process according to the invention can in principle be carried out in any reactor suitable for the respective reaction type. Without restricting generality, the following are mentioned by way of example: suspension reactor, stirred tank, stirred tank cascade, tubular reactor, shell-type reactor, shell and tube reactor, fixed-bed reactor, fluidized-bed reactor, reactive distillation column.

The following examples are provided to further illustrate the processes of the present invention. These examples are illustrative only and are not intended to limit the scope of the invention in any way.

### Examples

### Instruments and Materials

Nuclear magnetic resonance spectra were recorded on a Bruker Avance 300 spectrometer equipped with 5 mm BBO BB-1 H probe head operating at 300 MHz for ¹H and 75.5 MHz for ¹³C. Spectra were recorded in in DMSO-d6 and referenced to the residual solvent signal (¹H: 2.50 ppm).

Analytical U-HPLC chromatograms: measured on a Waters Acquity Ultra Performance Liquid Chromatography (UPLC), equipped with an Acquity HSS T3 100 Å 1.8µm 2.1 x50 mm2 analytical column and a PDA detector operating in the 200-400 nm wavelength range. H2O + 0.02 % TFA (A phase) and MeCN + 0.02 % TFA (B phase) were used as eluents, with a flow of 0.5 mL/min.

### Abbreviations

| | |
|---|---|
| δ | NMR chemical shift in ppm |
| d | doublet |
| DMSO | dimethylsulfoxide |
| 9 | gram |
| Hz | hertz |
| J | coupling constant in hertz |
| m | multiplett |
| M | molar / molarity |
| MHz | megahertz |
| min | minute(s) |
| mL | milliliter |
| mmol | millimole |
| ppm | parts per million |
| s | singlet |

### 1. Preparation of 2-chloro-1-(2,4-dihydroxyphenyl)ethan-1-one

To 4 M HCl in dioxane (17.0 mL, 68 mmol) at ambient temperature was added sequentially resorcinol (5 g, 45 mmol), ZnCl₂ (3.1 g 22.7 mmol) and 2-chloroacetonitrile (3.8 g, 50 mmol). The reaction was stirred for 3 h and then water (17 mL) was added, giving a thick suspension. The temperature was then increased to 70 °C and stirring was continued for 2 h, giving almost a solution. The reaction was allowed to cool to ambient temperature and was then cooled to 0 °C, resulting in the formation of a precipitate. The precipitate was isolated by filtration and washed with water (50 mL). Yield: 4.4 g (52% based on resorcinol).

1H NMR: δ 11.80-11.50 (br s, 1H), 10.90-10.30 (br s, 1H), 7.72 (d, J = 8.9 Hz, 1H), 6.39 (dd, J = 8.9,. 2,3 Hz, 1H), 6.33 (d, J = 2.3 Hz, 1H), 5.00 (s, 2H).

### 2. Preparation of N-(4-(2,4-dihydroxyphenyl)thiazol-2-yl)isobutyramide (thiamidol) from 2-chloro-1-(2,4-dihydroxyphenyl)ethan-1-one

### 2.1. EtOH - no base

2-Chloro-1-(2,4-dihydroxyphenyl)ethan-1-one (2.00 g, 10.7 mmol) was dissolved in EtOH (22 mL) and N-carbamothioylisobutyramide (1.72 g, 11.79 mmol) was added. The solution was heated to reflux and was stirred at this temperature for 6 h.
Ratio 6-hydroxybenzofuran-3(2*H*)-one/ thiamidol: <1:500 (UPLC, 216 nm)
Work-up: After cooling to ambient temperature, water (10 ml) was added over approx. 5 min, resulting in the formation of a white precipitate. The precipitate was isolated by filtration, washed with water and dried in a vacuum oven.
Yield: 2.23 g (66%, HCl salt, >97% purity at 216 nm).
1H NMR: δ 11.96-11.91 (br s, 1H), 9.5-7.5 ppm (br s, 2H), 7.40 (d, J = 8.30, 1H), 7.14 (s, 1H), 6.09-6.03 (m, 2H), 2.26-2.23 (m, 1H), 0.89 (s, 3H), 0.87 (s, 3H).

### 2.2. Acetonitrile - no base

2-Chloro-1-(2,4-dihydroxyphenyl)ethan-1-one (9.65 mmol) was dissolved in acetonitrile (20 mL) and N-carbamothioylisobutyramide (11.79 mmol) was added. The solution was heated to reflux for 15 h.
Ratio 6-hydroxybenzofuran-3(2*H*)-one/ thiamidol: <1:500 (UPLC, 216 nm)
Work-up: The suspension was cooled to ambient temperature and was filtered. The filter cake was rinsed once with 15 mL acetonitrile and then dried in a vacuum oven.
Yield: 2.5 g (83%, HCl salt, >95% purity at 216 nm).

### 2.3. Acetone - no base

2-Chloro-1-(2,4-dihydroxyphenyl)ethan-1-one (9.65 mmol) was dissolved in acetone (20 mL) and N-carbamothioylisobutyramide (11.79 mmol) was added. The solution was heated to reflux for 18 h.
Ratio 6-hydroxybenzofuran-3(2*H*)-one/ thiamidol: <1:500 (UPLC, 216 nm)
Work-up: The suspension was cooled to ambient temperature and was filtered. The filter cake was rinsed once with 15 mL acetonitrile and then dried in a vacuum oven.
Yield: 2.3 g (75%, HCl salt, >95% purity at 216 nm).

### 2.4. CPME- no base

2-Chloro-1-(2,4-dihydroxyphenyl)ethan-1-one (37.5 mmol) was dissolved in CPME (77 mL) and N-carbamothioylisobutyramide (39.4 mmol) was added. The solution was heated to reflux for 12 h.
Ratio 6-hydroxybenzofuran-3(2*H*)-one/ thiamidol: <1:500 (UPLC, 216 nm)
Work-up: The solution was cooled to ambient temperature and the suspension was filtered. The filter cake was washed twice with CPME and was dried in a vacuum oven.
Yield: 11 g (93%, HCl salt, >95% purity at 216 nm).

### 2.5. Dioxane - no base

2-Chloro-1-(2,4-dihydroxyphenyl)ethan-1-one (2.0 g, 9.6 mmol) was dissolved in dioxane (10 mL) and N-carbamothioylisobutyramide (1.55 g, 10.6 mmol) was added. The solution was heated to reflux and was stirred at this temperature for 15 h.
Ratio 6-hydroxybenzofuran-3(2*H*)-one/ thiamidol: approx. <1:500 (UPLC, 216 nm)
Work-up: The resulting suspension was cooled to ambient temperature and was filtered. The filter cake was dried in a vacuum oven.
Yield: 2.7 g (88%, HCl salt, >95% purity at 216 nm)

### 2.6. EtOH/H₂O - neutralization of acid as formed

2-Chloro-1-(2,4-dihydroxyphenyl)ethan-1-one (9.65 mmol) was dissolved in EtOH/H₂O 9:1 (20 mL) and N-carbamothioylisobutyramide (11.79 mmol) was added. The initial pH was approx. 3. The solution was heated to reflux followed by neutralization of the acid as formed (pH was maintained at approx. 3.5) by the addition of sat. aq. NaHCO₃.(9.5 mmol). Reaction time was 3.5 h.
Ratio 6-hydroxybenzofuran-3(2*H*)-one : thiamidol: < 1:500 (UPLC, 216 nm)
Work-up: The solution was cooled to ambient temperature, and water (15 ml) was added over approx. 5 min, resulting in the formation of a white precipitate. The precipitate was isolated by filtration, washed with water and dried in a vacuum oven.
Yield: 1.7 g (63%), >95% purity (UPLC, 216 nm)

### 2.7. EtOH - 1.5 eq. base initially added (comparative)

2-Chloro-1-(2,4-dihydroxyphenyl)ethan-1-one (1.00 g, 5.36 mmol) was dissolved in EtOH (11 mL) and N-carbamothioylisobutyramide (0.86 g, 5.90 mmol) was added followed by NaHCO₃ (0.68 g, 8.04 mmol). The pH of the mixture was approx. 7. The suspension was heated to reflux and stirred at this temperature for 3 h.
Ratio 6-hydroxybenzofuran-3(2*H*)-one/thiamidol: approx. 1:11.5.
Work-up: After cooling to ambient temperature, water (10 ml) was added over approx. 5 min, resulting in the formation of a white precipitate. The precipitate was isolated by filtration, washed with water and dried in a vacuum oven.
Yield: 1.10 g (65%, HCl salt, approx. 93% purity at 216 nm containing approx. 5-6% of 6-hydroxybenzofuran-3(2*H*)-one)

### 2.9. EtOH/H₂O - slight excess of base initially added (comparative)

2-Chloro-1-(2,4-dihydroxyphenyl)ethan-1-one (9.65 mmol) was dissolved in EtOH/H₂O 9:1 (20 mL) and N-carbamothioylisobutyramide (11.79 mmol) followed by 1M NaHCO₃ (10 mmol) were added. The pH was approx. 7. The solution was heated to reflux and was stirred at this temperature for 3 h.
Ratio 6-hydroxybenzofuran-3(2*H*)-one/thiamidol: approx. 3.5:1
Work-up: The solution was cooled to ambient temperature, and water (15 ml) was added over approx. 5 min, resulting in the formation of a white precipitate. The precipitate was isolated by filtration, washed with water and dried in a vacuum oven. Yield: n.a., <30%, purity.

### 3. Preparation of N-(4-(2,4-dihydroxyphenyl)thiazol-2-yl)isobutyramide (thiamidol) from resorcinol without isolation of2-chloro-1-(2,4-dihydroxyphenyl)ethan-1-one

### 3.1. CPME - no base

AlCl₃ (417 mmol) was dissolved in a solution of 150 mL CPME and 200 mL 4.9M HCl in CPME (980 mmol HCl). 2-Chloroacetonitrile (417 mmol) was added in one portion at 5 °C, then a solution of resorcinol (409 mmol) in 50 mL CPME was added over 10 min (rinsing with 20 mL CPME). The reaction was warmed to 25 °C and was stirred at that temperature for 16 h. 400 mL of water were then added over 80 min such that the internal temperature remained below 30 °C (bath temperature at 10 °C). The suspension was warmed to 60 °C and was stirred at that temperature for 1 h. The temperature was then increased to 65 °C, giving a solution. At 20 °C, 100 mL of water were added followed by adjusting the pH to 5 with aqueous NaOH. The organic layer was then washed twice with 200 mL of water and was then azeotropically dried at 200 mbar at 80 °C for 3h. To the resulting solution, containing approx. 278 mmol of 2-chloro-1-(2,4-dihydroxyphenyl)ethan-1-one (1H NMR analysis), was added a solution of N-carbamothioylisobutyramide (278 mmol) in 450 mL CPME. The solution was refluxed for 10 h with the removal of reaction water, and was then cooled to 10 °C.
Ratio 6-hydroxybenzofuran-3(2*H*)-one/ thiamidol: <1:500 (UPLC, 216 nm)
Work-up: 1M HCl in CPME (250 mmol) was added and the ensuing precipitate was isolated by filtration and was washed twice with 200 mL EtOAc.
Yield: 62.9 g (50% over two steps, HCl salt), >95% U-HPLC purity at 216 nm.

### 3.2. Dioxane - no base

To 36 mL 4M HCl in dioxane at 0 °C was added resorcinol (45 mmol), AlCl₃ (41 mmol) and 2-chloroacetonitrile (41 mmol). The solution was allowed to warm to ambient temperature in the ice bath and was stirred for a total of 3 h. 39 mL water were added over 15 min and the ensuing suspension was then stirred at 70 °C for 1 h and then at ambient temperature overnight. To the resulting mixture was added 39 mmol of N-carbamothioylisobutyramide followed by heating to reflux temperature for 4 h.
Ratio 6-hydroxybenzofuran-3(2*H*)-one/ thiamidol: approx. <1:500 (UPLC, 216 nm)
Work-up: The reaction was cooled to ambient temperature, was filtered and washed three times with 40 ml of water. The filter cake was dried in a vacuum oven.
Yield: 5.7 g (44% over two steps), >95% UPLC purity at 216 nm.

### 3.3. CPME - acid neutralised as formed

To a solution containing 107 mmol of 2-chloro-1-(2,4-dihydroxyphenyl)ethan-1-one in 180 mL CPME synthesized as described in 3.1. above, were added 109 mmol of N-carbamothioylisobutyramide in 45 mL CPME. The solution was heated to reflux. Once the collection of water in the Dean-Stark trap was observed, a solution of N,N-diisopropylethylamine (107 mmol) in 30 mL CPME was added over 75 min (rinsing with 40 mL CPME). Stirring was continued for 5 min, then 150 mL CPME were evaporated at reflux temperature. The reaction was cooled to 20 °C.
Ratio 6-hydroxybenzofuran-3(2*H*)-one/thiamidol: approx. 1:200 (UPLC, 216 nm)
Work-up: At 63 °C internal temperature 75 mL water were added. 125 mL 4M NaOH were then added at 20 °C. The layers were separated and the organic layer was extracted once with diluted NaOH. The combined aqueous layers were washed once with 100 mL CPME. The combined aqueous layers were diluted with 275 mL EtOH and the pH was adjusted to approx. 7 with 18.5% HCl. 200 mL water were added and the ensuing suspension was aged overnight at 20 °C. After cooling to 0 °C and adding additional 100 mL of water, the suspension was filtered. The filter cake was washed twice with H₂O/EtOH 2:1 and once with H₂O/EtOH 3:1, and was dried in a vacuum oven.
Yield: 23.7 g (52% over two steps), >95% U-HPLC purity at 216 nm.

### 2.14. Dioxane - acid neutralised as formed

To a solution containing 9.6 mmol of 2-chloro-1-(2,4-dihydroxyphenyl)ethan-1-one in 20 mL dioxane/H₂O 9:1 synthesized as described in 3.2., were added 10.6 mmol of N-carbamothioylisobutyramide. The solution was heated to reflux and the acid was neutralized as formed (pH was maintained at 3.0-4.5) by the addition of 2M NaOAc (10 mmol). After refluxing for 3 h, the reaction was cooled to 20 °C. At 85 °C internal temperature 10 mL water were added. At ambient temperature, 1 mL 37% HCl was added dropwise, the ensuing suspension was stirred overnight and was then filtered. The filter cake was washed twice with 15 mL H₂O. The filter cake was dried in a vacuum oven.
Yield: 1.9 g (HCl salt, 56% over two steps), >95% U-HPLC purity at 216 nm.

## Claims

1. A process for the preparation of a compound of formula (I) 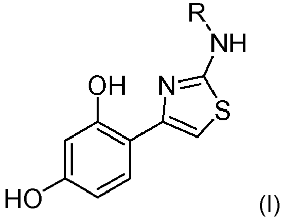 wherein
R is selected from the group of H or C(=O)R' with R' C₁₋₁₂ alkyl, hydroxyC₁₋₁₂ alkyl, C₁₋₄ alkoxyC₁₋₁₂ alkyl, aminoC₁₋₁₂ alkyl, C₂₋₁₂ alkenyl, C₃₋₈cycloalkyl, hydroxyC₁₋₃alkylC₃₋₈cycloalkyl and p-hydroxyC₁₋₃ alkylarylC₁₋₃ alkyl,
said process comprising the step of reacting a compound of formula (II) with a compound of formula (III) 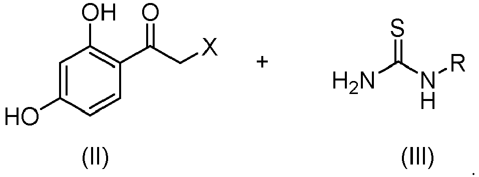
wherein
R is as defined above, and
X is a leaving group selected from Cl, Br, OTs and OMs, preferably CI, and
with the proviso that the reaction is either carried in the absence of a base or a base is continuously added such as to quench the acid (HX) formed during the reaction.

2. The process according to claim 1 wherein R is C(=O)R' with R' being a C₁₋₈ alkyl, more preferably a C₃₋₆ alkyl, most preferably isopropyl.

3. The process according to claim 1 or 2, wherein the reaction is carried out in an inert solvent.

4. The process according to claim 3, wherein the solvent is selected from the group consisting of water, alkyl alcohols, ethers and aromatic hydrocarbon solvents as well as any mixtures thereof.

5. The process according to claim 3 or 4, wherein the inert solvent is selected from the group consisting of ethanol, acetonitrile, acetone, dioxane and cyclopentyl methyl ether, optionally in admixture with water.

6. The process according to anyone of claims 1 to 5, wherein the reaction temperature is selected in the range from 40 C to 150°C, preferably in the range from 50°C to 110°C, more preferably in the range from 60° to 110, most preferably in the range of 70 to 110°C and/ or the reaction is maintained at reflux.

7. The process according to any one of claims 1 to 6, wherein the reaction time is selected in the range from 3 h to 24 h, more preferably in the range from 5 h to 22 h, most preferably in the range from 6 h to 10 h when no base is used or in the range from 30 min to 24 h, preferably in the range from 1 h to 12 h, most preferably in the range 1 h to 6 h when a base is used.

8. The process according to any one of claims 1 to 7, wherein the molar ratio of the compound of formula (III) to the compound of formula (II) is selected in the range from 1.5 to 1, preferably in the range from 1.25 to 1, most preferably in the range from 1.15 to 1.

9. The process according to any one of claims 3 to 8, wherein the amount of the inert solvent(s) is selected in the range from 0.25 to 15 mL/mmol of the compound of formula (II), preferably in the range from 0.5 to 10mL/mmol of the compound of formula (II), most preferably in the range from 1 to 5mL/mmol of the compound of formula (II).

10. The process according to anyone of the preceding claims, wherein the base is dosed such, that the pH is not exceeding pH 5.5.

11. The process according to anyone of the preceding claims, wherein the amount of base is selected in the range from 0.1 to 1.5 mole equivalents, preferably in the range from 0.25 to 1.25 mole equivalents, most preferably in the range from 0.5 to 1.15 mole equivalents, based on the compound of formula (II).

12. The process according to anyone of the preceding claims, wherein the base is selected from the group consisting of carbonates, preferably NaHCO₃ or from tertiary amine bases, preferably N,N-diisopropylethylamine and/ or 4-methylmorpholine.

13. The process according to anyone of the preceding claims, wherein the addition of base is performed after all reactants and optionally solvent(s) have been admixed and the resulting reaction mixture has been heated to at least 40°C, preferably at least 50°C, most preferably to reflux.

14. The process according to any one of the preceding claims, wherein the compound of formula (II) is 2-chloro-1-(2,4-dihydroxyphenyl)ethanone, preferably prepared from from resorcinol and chloroacetonitrile or from resorcinol and chloroacetylchloride.

15. The process according to anyone of the preceding claims, wherein the process comprises the steps
0-a) reacting resorcinol (IV) with chloroacetonitril (V) (Houben Hoesch reaction) in a solvent selected from cyclopentyl methyl ether or dioxane, followed by
0-b) hydrolysis of the resulting reaction mixture to obtain a compound of formula (Ila), 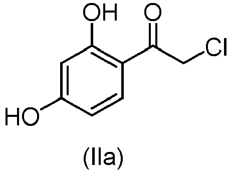 followed by
0-c) work-up and optional drying to obtain an organic phase comprising the solvent and the compound of formula (Ila),
followed by
1) addition of an acylthiourea of formula (IIIa) to the organic phase obtained in step 0-c)

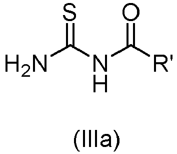

## Patentansprüche

1. Verfahren zur Herstellung einer Verbindung der Formel (I) 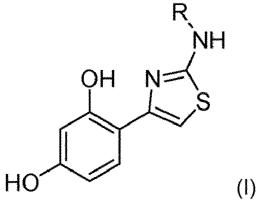 wobei
R aus der Gruppe von H oder C(=O)R' ausgewählt ist, wobei R' für C₁₋₁₂-Alkyl, Hydroxy-C₁₋₁₂-alkyl, C₁₋₄-Alkoxy-C₁₋₁₂-alkyl, Amino-C₁₋₁₂-alkyl, C₂₋₁₂-Alkenyl, C₃₋₈-Cycloalkyl, Hydroxy-C₁₋₃-alkyl-C₃₋₈-cycloalkyl und p-Hydroxy-C₁₋₃-alkylaryl-C₁₋₃-alkyl steht,
wobei das Verfahren den Schritt des Umsetzens einer Verbindung der Formel (II) mit einer Verbindung der Formel (III) 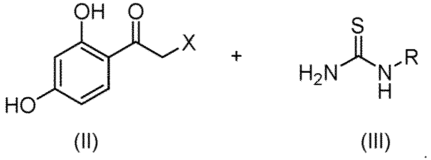
umfasst, wobei
R wie oben definiert ist und
X für eine Abgangsgruppe, die aus Cl, Br, OTs und OMs ausgewählt ist, vorzugsweise Cl, steht, und
mit der Maßgabe, dass die Umsetzung entweder in Abwesenheit einer Base durchgeführt wird oder eine Base kontinuierlich zugegeben wird, um die bei der Umsetzung gebildete Säure (HX) zu quenchen.

2. Verfahren nach Anspruch 1, wobei R für C(=O)R' steht, wobei R' für ein C₁₋₈-Alkyl, weiter bevorzugt ein C₃₋₆-Alkyl, ganz besonders bevorzugt Isopropyl, steht.

3. Verfahren nach Anspruch 1 oder 2, wobei die Umsetzung in einem inerten Lösungsmittel durchgeführt wird.

4. Verfahren nach Anspruch 3, wobei das Lösungsmittel aus der Gruppe bestehend aus Wasser, Alkylalkoholen, Ethern und aromatischen Kohlenwasserstofflösungsmitteln sowie beliebigen Mischungen davon ausgewählt wird.

5. Verfahren nach Anspruch 3 oder 4, wobei das inerte Lösungsmittel aus der Gruppe bestehend aus Ethanol, Acetonitril, Aceton, Dioxan und Cyclopentylmethylether, gegebenenfalls in Abmischung mit Wasser, ausgewählt wird.

6. Verfahren nach einem der Ansprüche 1 bis 5, wobei die Reaktionstemperatur im Bereich von 40 °C bis 150 °C, vorzugsweise im Bereich von 50 °C bis 110 °C, weiter bevorzugt im Bereich von 60 °C bis 110 °C, ganz besonders bevorzugt im Bereich von 70 bis 110 °C, gewählt wird und/oder der Ansatz am Rückfluss gehalten wird.

7. Verfahren nach einem der Ansprüche 1 bis 6, wobei die Reaktionszeit im Bereich von 3 h bis 24 h, weiter bevorzugt im Bereich von 5 h bis 22 h, ganz besonders bevorzugt im Bereich von 6 bis 10 h gewählt wird, wenn keine Base verwendet wird, oder im Bereich von 30 min bis 24 h, vorzugsweise im Bereich von 1 h bis 12 h, ganz besonders bevorzugt im Bereich von 1 h bis 6 h gewählt wird, wenn eine Base verwendet wird.

8. Verfahren nach einem der Ansprüche 1 bis 7, wobei das Molverhältnis der Verbindung der Formel (III) zu Verbindung der Formel (II) im Bereich von 1,5 zu 1, vorzugsweise im Bereich von 1,25 zu 1, ganz besonders bevorzugt im Bereich von 1,15 zu 1, gewählt wird.

9. Verfahren nach einem der Ansprüche 3 bis 8, wobei die Menge des inerten Lösungsmittels bzw. der inerten Lösungsmittel im Bereich von 0,25 bis 15 ml/mmol der Verbindung der Formel (II), vorzugsweise im Bereich von 0,5 bis 10 ml/mmol der Verbindung der Formel (II), ganz besonders bevorzugt im Bereich von 1 bis 5 ml/mmol der Verbindung der Formel (II), gewählt wird.

10. Verfahren nach einem der vorhergehenden Ansprüche, wobei die Base so dosiert wird, dass der pH-Wert 5,5 nicht überschreitet.

11. Verfahren nach einem der vorhergehenden Ansprüche, wobei die Basenmenge im Bereich von 0,1 bis 1,5 Moläquivalenten, vorzugsweise im Bereich von 0,25 bis 1,25 Moläquivalenten, ganz besonders bevorzugt im Bereich von 0,5 bis 1,15 Moläquivalenten, bezogen auf die Verbindung der Formel (II), gewählt wird.

12. Verfahren nach einem der vorhergehenden Ansprüche, wobei die Base aus der Gruppe bestehend aus Carbonaten, vorzugsweise NaHCO₃, oder aus tertiären Aminbasen, vorzugsweise N,N-Diisopropylethylamin und/oder 4-Methylmorpholin, ausgewählt wird.

13. Verfahren nach einem der vorhergehenden Ansprüche, wobei die Basenzugabe durchgeführt wird, nachdem alle Reaktanten und gegebenenfalls Lösungsmittel gemischt worden sind und die erhaltene Reaktionsmischung auf mindestens 40 °C, vorzugsweise mindestens 50 °C, ganz besonders bevorzugt zum Rückfluss, erhitzt worden ist.

14. Verfahren nach einem der vorhergehenden Ansprüche, wobei es sich bei der Verbindung der Formel (II) um vorzugsweise aus Resorcin und Chloracetonitril oder aus Resorcin und Chloracetylchlorid hergestelltes 2-Chlor-1-(2,4-Dihydroxyphenyl)ethanon handelt.

15. Verfahren nach einem der vorhergehenden Ansprüche, wobei das Verfahren die folgenden Schritte umfasst:
0-a) Umsetzen von Resorcin (IV) mit Chloracetonitril (V) (Houben-Hoesch-Reaktion) in einem aus Cyclopentylmethylether oder Dioxan ausgewählten Lösungsmittel, gefolgt von
0-b) Hydrolyse der erhaltenen Reaktionsmischung zum Erhalt einer Verbindung der Formel (IIa), 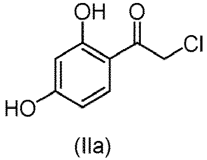 gefolgt von
0-c) Aufarbeitung und gegebenenfalls Trocknung zum Erhalt einer organischen Phase, die das Lösungsmittel und die Verbindung der Formel (IIa) umfasst,
gefolgt von
1) Zugabe eines Acylthioharnstoffs der Formel (IIIa) zu der in Schritt 0-c) erhaltenen organischen Phase

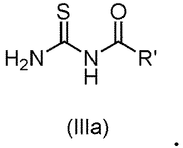

## Revendications

1. Procédé pour la préparation d'un composé de formule (I) 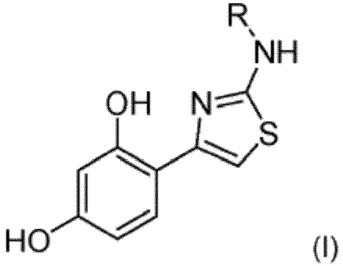
R étant choisi dans le groupe de H ou C(=O)R',
R' étant C₁₋₁₂ alkyle, hydroxyC₁₋₁₂ alkyle, C₁₋₄ alcoxyC₁₋₁₂ alkyle, aminoC₁₋₁₂ alkyle, C₂₋₁₂ alcényle, C₃₋₈cycloalkyle, hydroxyC₁₋₃alkylC₃₋₈cycloalkyle et p-hydroxyC₁₋₃ alkylarylC₁₋₃ alkyle,
ledit procédé comprenant l'étape de mise en réaction d'un composé de formule (II) avec un composé de formule (III) 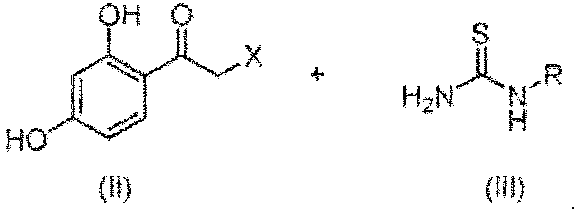
R étant tel que défini ci-dessus, et
X étant un groupe partant choisi parmi Cl, Br, OTs et OMs, préférablement Cl, et
à la condition que la réaction soit soit mise en œuvre en l'absence d'une base, soit qu'une base soit ajoutée de manière continue de sorte à piéger l'acide (HX) formé pendant la réaction.

2. Procédé selon la revendication 1, R étant C(=O)R', R' étant un C₁₋₈ alkyle, plus préférablement un C₃₋₆ alkyle, le plus préférablement isopropyle.

3. Procédé selon la revendication 1 ou 2, la réaction étant mise en œuvre dans un solvant inerte.

4. Procédé selon la revendication 3, le solvant étant choisi dans le groupe constitué par l'eau, des alcools alkyliques, des éthers et des solvants hydrocarbonés aromatiques ainsi que de quelconques mélanges correspondants.

5. Procédé selon la revendication 3 ou 4, le solvant inerte étant choisi dans le groupe constitué par l'éthanol, l'acétonitrile, l'acétone, le dioxanne et l'éther de cyclopentyle et de méthyle, éventuellement en mélange avec de l'eau.

6. Procédé selon l'une quelconque des revendications 1 à 5, la température de réaction étant choisie dans la plage de 40 C à 150 °C, préférablement dans la plage de 50 °C à 110 °C, plus préférablement dans la plage de 60° à 110, le plus préférablement dans la plage de 70 à 110 °C et/ou la réaction étant maintenue à reflux.

7. Procédé selon l'une quelconque des revendications 1 à 6, la durée de réaction étant choisie dans la plage de 3 h à 24 h, plus préférablement dans la plage de 5 h à 22 h, le plus préférablement dans la plage de 6 h à 10 h lorsqu'aucune base n'est utilisée ou dans la plage de 30 min à 24 h, préférablement dans la plage de 1 h à 12 h, le plus préférablement dans la plage de 1 h à 6 h lorsqu'une base est utilisée.

8. Procédé selon l'une quelconque des revendications 1 à 7, le rapport molaire du composé de formule (III) sur le composé de formule (II) étant choisi dans la plage de 1,5 à 1, préférablement dans la plage de 1,25 à 1, le plus préférablement dans la plage de 1,15 à 1.

9. Procédé selon l'une quelconque des revendications 3 à 8, la quantité du ou des solvants inertes étant choisie dans la plage de 0,25 à 15 mL/mmole du composé de formule (II), préférablement dans la plage de 0,5 à 10 mL/mmole du composé de formule (II), le plus préférablement dans la plage de 1 à 5 mL/mmole du composé de formule (II).

10. Procédé selon l'une quelconque des revendications précédentes, la base étant ajoutée de sorte que le pH ne dépasse pas pH 5,5.

11. Procédé selon l'une quelconque des revendications précédentes, la quantité de base étant choisie dans la plage de 0,1 à 1,5 équivalents en moles, préférablement dans la plage de 0,25 à 1,25 équivalent en moles, le plus préférablement dans la plage de 0,5 à 1,15 équivalent en moles, sur la base du composé de formule (II).

12. Procédé selon l'une quelconque des revendications précédentes, la base étant choisie dans le groupe constitué par des carbonates, préférablement NaHCO₃ ou parmi des bases de type amine tertiaire, préférablement la N,N-diisopropyléthylamine et/ou la 4-méthylmorpholine.

13. Procédé selon l'une quelconque des revendications précédentes, l'ajout de base étant réalisé après que tous les réactifs et éventuellement un ou des solvants ont été mélangés et que le mélange réactionnel résultant a été chauffé jusqu'à au moins 40 °C, préférablement au moins 50 °C, le plus préférablement jusqu'au reflux.

14. Procédé selon l'une quelconque des revendications précédentes, le composé de formule (II) étant la 2-chloro-1-(2,4-dihydroxyphényl)éthanone, préférablement préparée à partir de résorcinol et de chloroacétonitrile ou à partir de résorcinol et de chlorure de chloroacétyle.

15. Procédé selon l'une quelconque des revendications précédentes, le procédé comprenant les étapes
0-a) mise en réaction de résorcinol (IV) avec du chloroacétonitrile (V) (réaction de Houben Hoesch) dans un solvant choisi parmi l'éther de cyclopentyle et de méthyle ou le dioxanne, suivie par
0-b) hydrolyse du mélange réactionnel résultant pour obtenir un composé de formule (IIa), 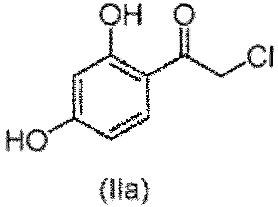 suivie par
0-c) traitement conclusif et éventuellement séchage pour obtenir une phase organique comprenant le solvant et le composé de formule (IIa),
suivi par
1) ajout d'une acylthiourée de formule (IIIa) à la phase organique obtenue dans l'étape 0-c)
